(19) Europäisches Patentamt
European Patent Office
Office européen des brevets

(11) Veröffentlichungsnummer: **0 153 907 B1**

# (12) EUROPÄISCHE PATENTSCHRIFT

(45) Veröffentlichungstag der Patentschrift: **11.11.92**

(51) Int. Cl.$^5$: **C07D 211/46**, C08K 5/34

(21) Anmeldenummer: **85810074.6**

(22) Anmeldetag: **22.02.85**

Die Akte enthält technische Angaben, die nach dem Eingang der Anmeldung eingereicht wurden und die nicht in dieser Patentschrift enthalten sind.

## (54) Verbindungen enthaltend Piperidinreste und ihre Verwendung zum Stabilisieren von synthetischen Polymeren.

(30) Priorität: **28.02.84 IT 1983084**

(43) Veröffentlichungstag der Anmeldung:
**04.09.85 Patentblatt 85/36**

(45) Bekanntmachung des Hinweises auf die Patenterteilung:
**11.11.92 Patentblatt 92/46**

(84) Benannte Vertragsstaaten:
**BE DE FR GB IT**

(56) Entgegenhaltungen:
**US-A- 4 369 321**

(73) Patentinhaber: **CIBA-GEIGY AG**
**Klybeckstrasse 141**
**CH-4002 Basel(CH)**
(84) Benannte Vertragsstaaten:
**BE DE FR GB**

(73) Patentinhaber: **CIBA-GEIGY S.p.A.**
**Strada Statale 233-Km. 20,5**
**Origgio(IT)**
(84) Benannte Vertragsstaaten:
**IT**

(72) Erfinder: **Cantatore, Giuseppe, Dr.**
**Via Generale Planelli, 68**
**I-70032 Bitonto (Bari)(IT)**
Erfinder: **Borzatta, Valerio, Dr.**
**Via Novelli 2**
**Bologna(IT)**

EP 0 153 907 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Piperidyloxyalkylamide, die zum Stabilisieren von synthetischen Polymeren gegen die Einwirkung von Licht, Wärme und Sauerstoff geeignet sind.

Es ist bekannt, dass die physikalischen und chemischen Eigenschaften von synthetischen Polymeren stark beeinträchtigt werden, wenn diese dem Sonnenlicht oder dem Licht einer anderen UV-Lichtquelle ausgesetzt werden. Zur Verbesserung der Lichtbeständigkeit dieser Polymeren wurden verschiedene Additive mit Lichtschutzwirkung vorgeschlagen. Diese bekannten Stabilisatoren vermögen aber nicht immer den heutigen Forderungen der Technik zu genügen. Eine weitere Verbesserung war demnach wünschenswert. Piperidylcarbamate analoger Struktur, die aber chemisch nicht mit den erfindungsgemässen Piperidyloxyalkylamiden vergleichbar sind, wurden in US- A 4 369 321 beschrieben. Die erfindungsgemässen Piperidyloxyalkylamide zeichnen sich durch ihre ausgezeichnete lichtstabilisierende Wirkung in Polymeren, insbesondere in Polyolefinen aus.

Die vorliegende Erfindung betrifft demnach Verbindungen der Formel I

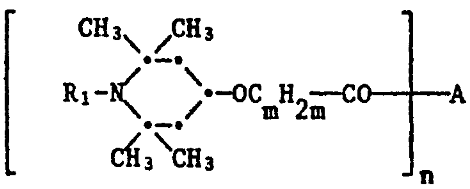

(I)

worin $R_1$ Wasserstoff, O , Cyanomethyl, $C_1$-$C_{12}$-Alkyl, $C_3$-$C_{12}$-Alkenyl oder Alkynyl, $C_7$-$C_{12}$-Aralkyl, Methylbenzyl, t-Butylbenzyl, Hydroxybenzyl oder $C_1$-$C_{12}$-Acyl ist, m eine ganze Zahl von 1 bis 12 und n eine ganze Zahl von 1 bis 3 bedeuten und A, wenn n = 1, eine Gruppe-N($R_2$)($R_3$) bedeutet, worin $R_2$ und $R_3$ unabhängig voneinander Wasserstoff, $C_1$-$C_{18}$-Alkyl, 2-Methoxyethyl, 2-Ethoxyethyl, 3-Methoxypropyl, 3-Ethoxypropyl, 3-Butoxypropyl, 3-Octyloxypropyl, 3-Dodecyloxypropyl, 3-Dimethylaminopropyl, 3-Diethylaminopropyl, 4-Diethylaminobutyl, $C_3$-$C_{18}$-Alkenyl, $C_5$-$C_{18}$-Cycloalkyl, Methylcyclohexyl, Trimethylcyclohexyl, $C_6$-$C_{18}$-Aryl, Methylphenyl, Dimethylphenyl, Tri-methylphenyl, t-Butylphenyl, Methoxyphenyl, Ethoxyphenyl, 3,5-Di-tert-butyl-4-hydroxyphenyl, $C_7$-$C_{18}$ Aralkyl, Methylbenzyl, Hydroxybenzyl, 3,5-Di-tert-butyl-4-hydroxybenzyl oder eine Gruppe der Formel II,

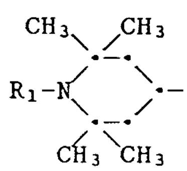

(II)

worin $R_1$ die oben angegebene Bedeutung hat, oder A Pyrrolidin-1-yl, Piperidin-1-yl, 2,2,6,6-Tetramethylpiperidin-1-yl, Morpholin-4-yl, 4-Methyl-piperazin-1-yl, 3,3,5,5-Tetramethyl-piperazin-1-yl, Hexahydroazepin-1-yl, Azacyclotridec-1-yl oder einen Rest der Formel

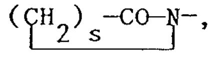

worin s eine ganze Zahl von 3 bis 11 ist, bedeutet und A, wenn n = 2, einen Rest der Formel III, IV oder V

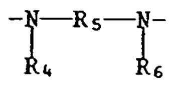
(III)
,
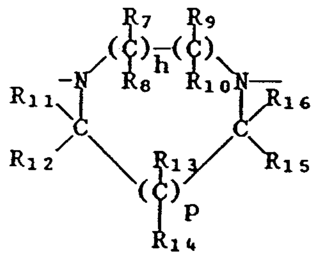
(IV)
,
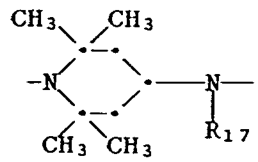
(V)

bedeutet, worin $R_4$, $R_6$ und $R_{17}$ unabhängig voneinander die bereits für $R_2$ und $R_3$ angegebene Bedeutung haben, $R_5$ $C_2$-$C_{18}$-Alkylen, 3-Oxapentan-1,5-diyl, 4-Oxaheptan-1,7-diyl, 4,9-Dioxadodecan-1,12-diyl, 4,7,10-Trioxatridecan-1,13-diyl, $C_5$-$C_{12}$-Cycloalkylen, Cyclohexylen-dimethylen, $C_6$-$C_{12}$-Arylen, Xylylen oder $C_7$-$C_{12}$-Aralkylen ist, die Reste $R_7$ bis $R_{16}$ unabhängig voneinander Wasserstoff oder Methyl bedeuten, $R_9$ zusammen mit $R_{10}$ und $R_{13}$ zusammen mit $R_{14}$ auch Sauerstoff bedeuten können, p und h unabhängig voneinander Null oder 1 sind, und

A, wenn n = 3, eine Gruppe der Formel VI

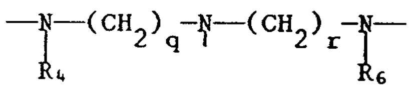
(VI)

bedeutet, worin $R_4$ und $R_6$ die oben angegebene Bedeutung haben und q und r unabhängig voneinander eine ganze Zahl von 2 bis 6 sind oder A einen Hexahydro-1,3,5-triazin-1,3,5-triyl-Rest bedeutet.

Die Bedeutungen der verschiedenen Reste speziell erläuternde Beispiele sind wie folgt:

Für $R_1$: Wasserstoff, Cyanomethyl, Methyl, Ethyl, Propyl, Butyl, Hexyl, Octyl, Decyl, Dodecyl, Allyl, Methallyl, 2-Butenyl, 2-Hexenyl, 10-Undecenyl, Propargyl, Benzyl, Methylbenzyl, t-Butylbenzyl, Hydroxybenzyl, Acetyl, Propionyl, Butyryl, Caproyl, Benzoyl, Acryloyl, Methacryloyl und Crotonyl;

Für $R_2$, $R_3$, $R_4$, $R_6$ und $R_{17}$: Wasserstoff, Methyl, Ethyl, Propyl, Isopropyl, Butyl, Isobutyl, Hexyl, Heptyl, Octyl, 2-Ethylhexyl, 1,1,3,3-Tetramethylbutyl, Decyl, Dodecyl, Hexadecyl, Octadecyl, 2-Methoxyethyl, 2-Ethoxyethyl, 3-Methoxypropyl, 3-Ethoxypropyl, 3-Butoxypropyl, 3-Octyloxypropyl, 3-Dodecyloxypropyl, 3-Dimethylaminopropyl, 3-Diethylaminopropyl, 4-Diethylaminobutyl, Allyl, Methallyl, 2-Butenyl, 2-Hexenyl, 10-Undecenyl, Oleyl, Cyclohexyl, Methylcyclohexyl, Trimethylcyclohexyl, Cyclooctyl, Cyclododecyl, Phenyl, Methylphenyl, Dimethylphenyl, Tri-methylphenyl, t-Butylphenyl, Methoxyphenyl, Ethoxyphenyl, 3,5-Di-t-butyl-4-hydroxyphenyl, Benzyl, Methylbenzyl, Hydroxybenzyl, 3,5-Di-t-butyl-4-hydroxybenzyl, 2,2,6,6-Tetramethylpiperidin-4-yl, 1,2,2,6,6-Pentamethyl-piperidin-4-yl, 1-Allyl-2,2,6,6-tetramethyl-piperidin-4-yl, 1-Benzyl-2,2,6,6-tetramethyl-piperidin-4-yl und 1-Acetyl-2,2,6,6-tetramethyl-piperidin-4-yl;

Für $R_5$: Ethylen, 1,2-Propylen, Trimethylen, Pentamethylen, Hexamethylen, Decamethylen, Dodecamethylen Cyclohexylen, Cyclohexylendimethylen, Phenylen, Xylylen, 3-Oxapentan-1,5-diyl, 4-Oxaheptan-1,7-diyl, 4,9-Dioxadodecan-1,12-diyl und 4,7,10-Trioxatridecan-1,13-diyl.

Für A als zweiwertiger Rest der Formel IV:

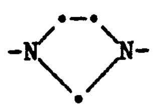 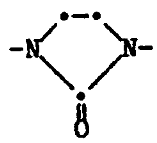 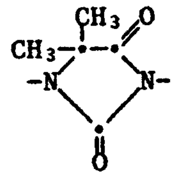 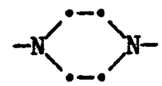

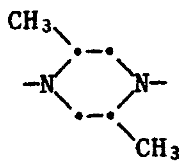 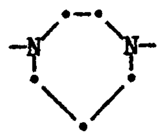 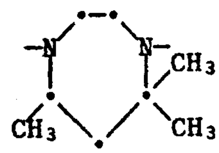

Wenn n = 3 ist, bedeutet A bevorzugt Hexahydro-1,3,5-triazin-1,3,5-triyl.

Bevorzugt werden Verbindungen der Formel (I), worin $R_1$ Wasserstoff, Methyl, Allyl, Benzyl oder Acetyl bedeutet, m eine Zahl von 1 bis 6 ist, $R_2$, $R_3$, $R_4$, $R_6$ und $R_{17}$ Wasserstoff, $C_1$-$C_{12}$-Alkyl, 3-($C_1$-$C_6$-Alkoxy)-propyl, $C_6$-$C_{12}$-Cycloalkyl oder eine Gruppe der Formel II worin $R_1$ die bereits für diese Bevorzugung angegebene Bedeutung hat und $R_5$ $C_2$-$C_{12}$-Alkylen ist oder A als heterocyclischer Rest Piperidin-1-yl, 2,2,6,6-Tetramethyl-piperidin-1-yl, Hexahydroazepin-1-yl, Morpholin-4-yl, Piperazin-1,4-diyl oder 5,5,7-Trimethylhomopiperazin-1,4-diyl bedeutet.

Besonders bevorzugt werden Verbindungen der Formel I, worin $R_1$ Wasserstoff oder Methyl, m 1 oder 2, $R_2$, $R_3$, $R_4$, $R_6$ und $R_{17}$ $C_1$-$C_8$-Alkyl, Cyclohexyl, 2,2,6,6-Tetramethyl-piperidin-4-yl oder 1,2,2,-6,6-Pentamethyl-piperidin-4-yl und $R_5$ $C_2$-$C_6$-Alkylen bedeuten oder A als heterocyclischer Rest Piperazin-1,4-diyl oder 5,5,7-Trimethylhomopiperazin-1,4-diyl ist.

Die neuen erfindungsgemässen Verbindungen können wie folgt hergestellt werden:

a) Umestzung eines Halogenamids der Formel VII

$A\text{-}(COC_mH_{2m}X)_n$ (VII)

worin A, m und n die oben angegebene Bedeutung haben und X Chlor oder Brom ist, mit einem Alkoholat der Formel VIII

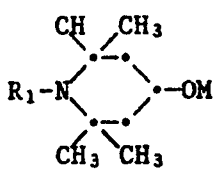

(VIII)

worin $R_1$ die oben angegebene Bedeutung hat und M ein Alkalimetall ist, in einem inerten organischen Lösungsmittel bei Temperaturen zwischen 0 und 200 °C, insbesondere zwischen 20 und 150 °C, vorzugsweise im stöchiometrischen Verhältnis;

b) Umsetzung eines Halogenamids der Formel VII mit einem Piperidinol der Formel IX

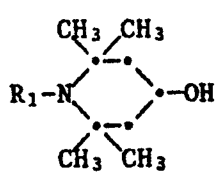

(IX)

worin $R_1$ die oben angegebene Bedeutung hat, in einem inerten organischen Lösungsmittel bei Temperaturen zwischen 20 und 200 °C, insbesondere zwischen 40 und 150 °C, vorzugsweise im

stöchiometrischen Verhältnis, in Gegenwart einer anorganischen Base, wie z.B. Natrium- oder Kaliumhydroxyd oder -carbonat, und eines Katalysators der Formel $(R_aR_bR_cR_dY)^+Z^-$, worin $R_a$, $R_b$, $R_c$ und $R_d$ unabhängig voneinander $C_1$-$C_{18}$-Alkyl, $C_6$-$C_9$-Aryl oder $C_7$-$C_{18}$-Aralkyl bedeuten, Y Stickstoff oder Phosphor ist und $Z^-$ ein Anion, wie z.B. $Cl^-$, $Br^-$, $I^-$, $OH^-$ oder $HSO_4{}^-$ bedeutet, oder in Gegenwart eines Katalysators der Formel X

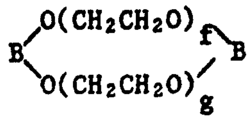

(X),

worin B $-CH_2CH_2-$,

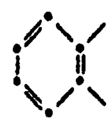

oder

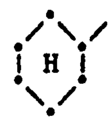

bedeutet, und f und g unabhängig voneinander eine Zahl von 1 bis 3 sind,
oder in Gegenwart eines Katalysators der Formel XI

$HO(CH_2CH_2O)_tH$ (XI)

worin t eine Zahl zwischen 4 und 250 bedeutet,
oder in Gegenwart einer festen anorganischen Base, wie Natrium- oder Kaliumhydroxyd, ohne den Zusatz eines organischen Katalysators.

Als Katalysatoren können beispielsweise verwendet werden: Tetraethylammoniumbromid, Tetrabutylammoniumbromid, Tetrabutylammoniumhydrogensulfat, Benzyltriethylammoniumchlorid, Benzyltriethylammoniumbromid, Lauryldimethylbenzylammoniumchlorid, Cetyltrimethylammoniumbromdid, Myristyltrimethylammoniumbromid, Benzyltriphenylphosphoniumchlorid, Kronenether wie z.B. 18-Crown-6-, 15-Crown-5- oder übliche Polyethylenglykole.

Die Halogenamide der Formel VII können in Analogie zu allgemein bekannten Methoden hergestellt werden, wie z.B. durch Umsetzung eines Halogenacylhalogenids $XCOC_mH_{2m}X$ mit einem Amin der Formel $A\text{-}(H)_n$, worin A, X, m und n die oben angegebene Bedeutung haben, bei tiefer Temperatur, vorzugsweise unter 0°C, in einem geeigneten Lösungsmittel, beispielsweise einem chlorierten Kohlenwasserstoff, und anschliessender Zugabe der entsprechenden stöchiometrischen Menge NaOH. Die Halogenamide können direkt ohne Isolierung vom Reaktionsgemisch oder nach Abtrennung und Reinigung für die weitere Umsetzung eingesetzt werden.

Wie anfangs erwähnt, sind die Verbindungen der Formel I sehr wirksam bei der Verbesserung der Licht-, Wärme- und Oxydationsbeständigkeit von synthetischen Polymeren wie beispielsweise Polyethylen hoher oder niedriger Dichte, Polypropylen, Ethylen/Propylencopolymer, Ethylen/Vinylacetatcopolymeren, Polybutadien, Polyisopren, Polystyrol, Butadien/Styrolcopolymeren, Acrylnitril/Butadien/Styrolco-polymeren, Polymeren und Copolymeren von Vinylchlorid und Vinylidenchlorid, Polyoxymethylen, Polyurethanen, gesättigten und ungesättigten Polyestern, Polyamiden, Polycarbonaten, Polyacrylaten, Alkydharzen und Epoxidharzen.

Die Verbindungen der Formel I können im Gemisch mit synthetischen Polymeren in verschiedenen Anteilen eingesetzt werden, die von der Art des Polymeren, dem Endzweck und der Gegenwart weiterer Zusatzstoffe abhängen. Im allgemeinen setzt man zweckmässig 0,01 bis 5 Gew.-% der Verbindungen der Formel I, bezogen auf das Gewicht der Polymeren, vorzugsweise 0,05 bis 1 %, ein.

Die Verbindungen der Formel I können nach verschiedenen Verfahren in die polymeren Materialien eingearbeitet werden, wie durch trockenes Einmischen in Pulverform oder nasses Einmischen in Form einer Lösung oder Suspension oder auch in Form eines konzentrierten Vorgemisches; das synthetische Polymer lässt sich dabei in Form von Pulver, Granulat, Lösung, Suspension oder Latex einsetzen. Die mit den Produkten der Formel I stabilisierten Polymeren lassen sich zur Herstellung von Formkörpern, Folien, Bändern, Fasern, Endlosfäden, Lacken usw. verwenden.

Gegebenenfalls kann man den Mischungen von Verbindungen der Formel I mit den synthetischen

Polymeren weitere Zusatzstoffe beigeben, wie beispielsweise Antioxydantien, UV-absorbierende Stoffe, Nickelstabilisatoren, Pigmente, Füllstoffe, Weichmacher, Antistatika, Flammschutzmittel, Schmierstoffe, Korrosionshemmstoffe sowie Metalldesaktivatoren. Beispiele für in Mischung mit Verbindungen der Formel I verwendbare Zusatzstoffe sind insbesondere:

1. Antioxidantien

1.1. Alkylierte Monophenole

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-($\alpha$-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone

2,6-Di-tert.butyl-4-methoxyphenol
2,5-Di-tert.butyl-hydrochinon
2,Di-tert.amyl-hydrochinon
2,6-Diphenyl-4-octadecyloxyphenol

1.3. Hydroxylierte Thiodiphenylether

2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

1.4. Alkyliden-Bisphenole

2,2'-Methylen-bis-(6-tert.butyl-4-methylphenol)
2,2'-Methylen-bis-(6-tert.butyl-4-ethylphenol)
2,2'-Methylen-bis-[4-methyl-6($\alpha$-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-($\alpha$-methylbenzyl)-4-nonylphenol]
2,2'-Methlyen-bis-[6-($\alpha$,$\alpha$-dimethylbenzyl)-4-nonylphenol]
4,4'-Methylen-bis-(2,6-di-tert.butylphenol)
4,4'-(Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

1.5 Benzylverbindungen

1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz.

1.6. Acylsminophenole

4-Hydroxy-laurinsäureanilid

4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit

| | |
|---|---|
| Methanol<br>Octadecanol<br>1,6-Hexandiol<br>Neopentylglycol<br>Thiodiethylenglycol | Diethylenglycol<br>Triethylenglycol<br>Pentaerythrit<br>Tris-hydroxyethyl-isocyanurat<br>Di-hydroxyethyl-oxalsäurediamid |

1.8. Ester der β-(5-tert.butyl-4-hydroxy-3-methylphenyl)-propionsäure mit ein- oder mehrwertigen Alkoholen, wie z.B. mit

| | |
|---|---|
| Methanol<br>Octadecanol<br>1,6-Hexandiol<br>Neopentylglycol<br>Thiodiethylenglycol | Diethylenglycol<br>Triethylenglycol<br>Pentaerythrit<br>Tris-hydroxyethyl-isocyanurat<br>Di-hydroxyethyl-oxalsäurediamid |

1.9. Amide der β-(3,5-Di-tert.butyl-4-hydroxyphenyl)-propionsäure, wie z.B.
N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N<N'-Di-(3,5-di-tert.butyl-4-hydroxyphenylpropionyl)-hydrazin

2. UV-Absorber und Lichtschutzmittel

2.1. 2-(2'-Hydroxyphenyl)-benztriazole, wie z.B. das 5'-Methyl-, 3',5'-Di-tert.butyl-, 5'-Tert.butyl-, 5'-(1,1,3,3-Tetramethylbutyl)-,5-Chlor-3',5'-di-tert.butyl-, 5-Chlor-3'-tert.butyl-5'-methyl-,3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, a-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin,

2.5. Nickelverbindungen, wie z.B. Nickelkomplexe des 2,2'-Thio- bis -[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.Octylamino-2,6-dichlor-1,3,5-s-triazin,
Tris-(2,2,6,6-tetramethyl-4-piperidyl)-nitrilotriacetat,
Tetrakis-(2,2,6,6-tetramethyl-4-piperidyl)-1,2,3,4-butantetrscarbonsäure,
1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho-und para-Methoxy- sowie von o- und p-Ethoxy-di-substituierte Oxaniliden.

3. Metalldesaktivatoren, wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Die folgenden Beispiele sollen der besseren Veranschaulichung der vorliegenden Erfindung dienen und bedeuten keinerlei Einschränkung der Erfindung.

Beispiel 1: N,N'-Bis-[2-(1,2,2,6,6-Pentamethylpiperidin-4-yloxy)-acetyl]-N,N'-bis-(2,2,6,6-tetramethyl-piperidin-4-yl)-1,6-diaminohexan

38,9 g (0,07 Mol) N,N'-Bis-(2-chloracetyl)-N,N'-bis-(2,2,6,6-tetramethyl-piperidin-4-yl)-1,6-diaminohexan werden in 100 ml wasserfreiem Xylol gelöst. Die erhaltene Lösung wird auf 0 bis 10 °C gekühlt und darin wird eine zuvor durch Umsetzung von 3,54 g (0,154 Mol) Natrium mit 26,38 g (0,154 Mol) 1,2,2,6,6-Pentamethylpiperidin-4-olin 120 ml wasserfreiem Xylol erhaltene Lösung des Na-Alkoholats von 1,2,2,6,6-Pentamethyl-piperidin-4-ol bei ständigem Rühren langsam zugetropft. Die Temperatur muss während der Zugabe zwischen 0 und 10 °C gehalten werden. Nach Beendigung der Zugabe rührt man das Reaktionsgemisch 2 Stunden bei Zimmertemperatur und weitere 8 Stunden bei 60-70 °C.

Die Lösung wird dann abgekühlt, mit Wasser gewaschen, getrocknet und die flüchtigen Anteile vollständig abgedampft. Der ölige Rückstand wird in n-Hexan kristallisiert und ergibt ein Produkt mit Smp. 116,8 °C.

| Analyse für $C_{48}H_{92}N_6O_4$ | | | |
|---|---|---|---|
| Berechnet: | C 70,54 % | H 11,35 % | N 10,28 % |
| Gefunden: | C 70,64 % | H 11,43 % | N 10,33 % |

Beispiele 2-13: Wiederholt man das unter Beispiel 1 beschriebene Verfahren mit entsprechenden Ausgangsprodukten, so erhält man folgende Verbindungen der Formel I

Tabelle 1

| Beisp. Nr. | $R_1$ | m | n | A | Smp. (°C) |
|---|---|---|---|---|---|
| 2 | H | 1 | 2 | 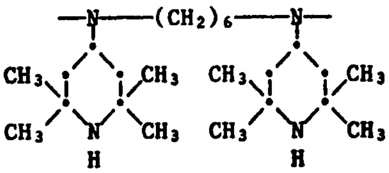 | 44-45 |
| 3 | $CH_3$ | 1 | 2 | 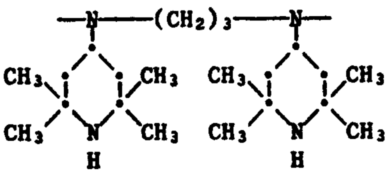 | 116-117 |
| 4 | H | 1 | 2 | 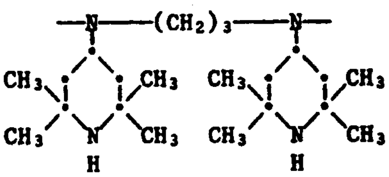 | 95-96 |
| 5 | $CH_3$ | 1 | 2 | 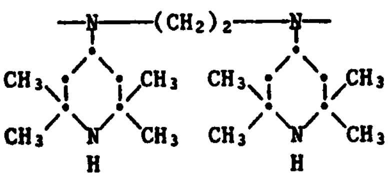 | 166-167 |
| 6 | H | 1 | 2 | 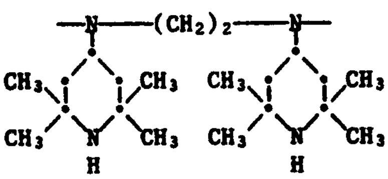 | 180-181 |
| 7 | $CH_3$ | 1 | 2 | 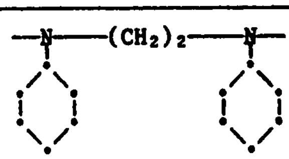 | 150-151 |
| 8 | H | 1 | 2 | 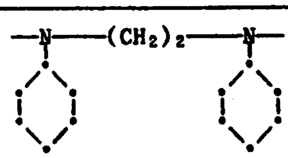 | 183-184 |

**Tabelle 1 (Fortsetzung)**

| Beisp. Nr. | $R_1$ | m | n | A | Smp. (°C) |
|---|---|---|---|---|---|
| 9 | $CH_3$ | 2 | 2 | 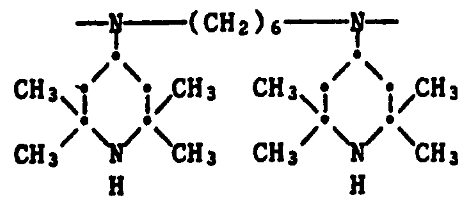 | 77-78 |
| 10 | $CH_3$ | 1 | 2 | 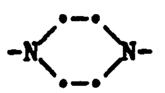 | 180-181 |
| 11 | H | 1 | 2 | 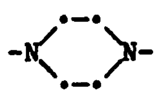 | 167-168 |
| 12 | $CH_3$ | 1 | 2 | 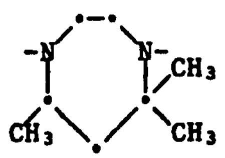 | 118-119 |
| 13 | H | 1 | 2 | 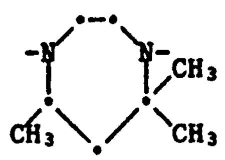 | 44-46 |
| 14 | $CH_3$ | 1 | 2 | 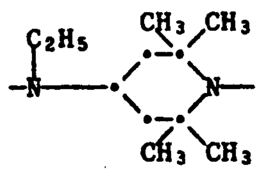 | Harz |

Beispiel 15: N-[2-(1,2,2,6,6-Pentamethyl-piperidin-4-yloxy)-acetyl]-N-(2,2,6,6-tetramethyl-piperidin-4-yl)-ethylamin

a) N-(2-Chloracetyl)-N-(2,2,6,6-tetramethylpiperidin-4-yl)-ethylamin:
Eine Lösung von 23,16 g (0,205 Mol) Chloracetylchlorid in 30 ml Methylenchlorid wird langsam unter Rühren einer auf -20 °C gekühlten Lösung von 36,85 g (0,2 Mol) N-(2,2,6,6-Tetramethyl-piperidin-4-yl)-ethylamin in 160 ml Methylenchlorid zugetropft, wobei die Temperatur -10 °C nicht übersteigen soll. Das Reaktionsgemisch wird eine Stunde weiter gerührt und dann stehen gelassen bis die Temperatur 0 °C erreicht. Danach wird eine Lösung von 8,4 g (0,21 Mol) NaOH in 35 ml Wasser während einer Stunde zugetropft. Das Reaktionsgemisch wird eine weitere Stunde bei Zimmertemperatur gerührt, die wässrige Phase abgetrennt und die organische Phase zur Trockne abgedampft.

Der Rückstand; der gemäss gaschromatographischer Analyse 98,7 % rein ist, kann in der rohen Form oder nach Umkristallisation aus n-Hexan weiter verwendet werden. Das kristalline Produkt schmilzt bei 58,5 °C.

| Analyse für $C_{13}H_{25}ClN_2O$ | | | | |
|---|---|---|---|---|
| Berechnet: | C 59,87 % | H 9,66 % | N 10,74 % | Cl 13,59 % |
| Gefunden: | C 59,93 % | H 9,78 % | N 10,70 % | Cl 13,50 % |

b) N-[2-(1,2,2,6,6-Pentamethyl-piperidin-4-yloxy)-acetyl]-N-(2,2,6,6-tetramethylpiperidin-4-yl)-ethylamin:
26,05 g (0,1 Mol) N-(2-Chloracetyl)-N-(2,2,6,6-tetramethyl-piperidin-4-yl)-ethylamin gemäss a) werden in 100 ml wasserfreiem Xylol gelöst. Die Lösung wird auf 0-10 °C gekühlt und darin wird eine zuvor durch Umsetzung von 2,53 g (0,11 Mol) Natrium mit 18,85 g (0,11 Mol) 1,2,2,6,6-Pentamethylpiperidin-4-ol in 85 ml wasserfreiem Xylol erhaltene Lösung des Na-Alkoholats von 1,2,2,6,6-Pentamethylpiperidin-4-ol bei ständigem Rühren langsam zugetropft. Die Temperatur muss während der Zugabe zwischen 0 und 10 °C gehalten werden. Nach Beendigung der Zugabe rührt man das Reaktionsgemisch 2 Stunden bei Zimmertemperatur und weitere 8 Stunden bei 60-70 °C.

Das Gemisch wird abgekühlt, mit Wasser gewaschen, getrocknet und die flüchtigen Anteile vollständig abgedampft. Der ölige Rückstand wird destilliert. Das Produkt siedet bei 193 °C/0,9 mbar und kristallisiert beim Stehenlassen bei Raumtemperatur. Smp. 66-67 °C.

| Analyse für $C_{23}H_{45}N_3O_2$ | | | |
|---|---|---|---|
| Berechnet: | C 69,83 % | H 11,46 % | N 10,62 % |
| Gefunden: | C 70,16 % | H 11,55 % | N 10,70 % |

Beispiel 16: N-[2-(2,2,6,6-Tetramethyl-piperidin-4-yloxy)-acetyl]-N-(2,2,6,6-tetramethyl-piperidin-4-yl)-ethylamin

Das obengenannte Produkt erhält man, wenn man in Analogie zu dem in Beispiel 15 beschriebenen Verfahren 26,05 g (0,1 Mol) N-(2-Chloracetyl)-N-(2,2,6,6-tetramethyl-piperidin-4-yl)-ethylamin mit 19,83 g (0,11 Mol) des Na-Alkoholats von 2,2,6,6-Tetramethyl-piperidin-4-ol in 85 ml Xylol umsetzt.

Durch Umkristallisieren des Rohprodukts aus n-Hexan erhält man das reine Produkt mit Smp. 75-76 °C.

| Analyse für $C_{22}H_{43}N_3O_2$ | | | |
|---|---|---|---|
| Berechnet: | C 69,24 % | H 11,36 % | N 11,01 % |
| Gefunden: | C 68,91 % | H 11,37 % | N 10,97 % |

Beispiel 17: N-[2-(1,2,2,6,6-Pentamethyl-piperidin-4-yloxy)-acetyl]-N-[2,2,6,6-tetramethyl-piperidin-4-yl)-butylamin

a) N-(2-Chloracetyl)-N-(2,2,6,6-tetramethyl-piperidin-4-yl)-butylamin:
Dieses Zwischenprodukt erhält man in Analogie zu dem unter Beispiel 15a beschriebenen Verfahren durch Umsetzung von 23,16 g (0,205 Mol) Chloracetylchlorid mit 42,48 g (0,2 Mol) N-(2,2,6,6-Tetramethyl-piperidin-4-yl)-butylamin.

Das reine Produkt erhält man durch Destillation
(Sdp. 138-139 °C/0,13 mbar)

| Analyse für $C_{15}H_{29}ClN_2O$ | | | | |
|---|---|---|---|---|
| Berechnet: | C 62,37 % | H 10,12 % | N 9,70 % | Cl 12,27 % |
| Gefunden: | C 62,45 % | H 10,18 % | N 9,61 % | Cl 12,19 % |

b) N-[2-(1,2,2,6,6-Pentamethyl-piperidin-4-yloxy)-acetyl]-N-(2,2,6,6-tetramethyl-piperidin-4-yl)-butylamin:
Dieses Produkt erhält man in Analogie zu dem unter Beispiel 15b beschriebenen Verfahren, durch Umsetzung von 28,8 g (0,1 Mol) N-(2-Chloracetyl)-N-(2,2,6,6-tetramethyl-piperidin-4-yl)-butylamin mit 21,23 g (0,11 Mol) des Na-Alkoholats von 1,2,2,6,6-Pentamethyl-piperidin-4-ol in 85 ml Xylol.

Das reine Produkt erhält man durch Umkristallisieren des Rohproduktes aus n-Hexan. Smp` 71-72 ° C.

| Analyse für $C_{25}H_{49}N_3O_2$ | | | |
|---|---|---|---|
| Berechnet: | C 70,87 % | H 11,66 % | N 9,92 % |
| Gefunden: | C 71,27 % | H 11,67 % | N 9,98 % |

Beispiel 18: N-[2-(2,2,6,6-Tetramethyl-piperidin-4-yloxy)-acetyl]-N-(2,2,6,6-tetramethyl-piperidin-4-yl)-butylamin

Dieses Produkt erhält man in Analogie zu dem unter Beispiel 15b beschriebenen Verfahren, durch Umsetzung von 28,8 g (0,1 Mol) N-(2-Chloracetyl)-N-(2,2,6,6-tetramethyl-piperidin-4-yl)-butylamin mit 19,83 g (0,11 Mol) des Na-Alkoholats von 2,2,6,6-Tetramethyl-piperidin-4-ol in 85 ml Xylol.

Das erhaltene Produkt siedet bei 179-180 ° C/0,65 mbar

| Analyse für $C_{24}H_{47}N_3O_2$ | | | |
|---|---|---|---|
| Berechnet: | C 70,37 % | H 11,56 % | N 10,26 % |
| Gefunden: | C 70,80 % | H 11,60 % | N 10,35 % |

Beispiel 19: N-[2-(2,2,6,6-Tetramethyl-piperidin-4-yloxy)-acetyl]-N-(2,2,6,6-tetramethyl-piperidin-4-yl)-3-methoxypropylamin

a) N-(2-Chloracetyl)-N-(2,2,6,6-tetramethyl-piperidin-4-yl)-methoxypropylamin:
Dieses Zwischenprodukt erhält man in Analogie zu dem unter Beispiel 15a beschriebenen Verfahren durch Umsetzung von 23,16 g (0,205 Mol) Chloracetylchlorid mit 45,68 g (0,2 Mol) N-2,2,6,6-Tetramethyl-piperidin-4-yl)-3-methoxypropylamin.

Das reine Produkt erhält man durch Destillation
(Sdp. 171-173 ° C/1,43 mbar).

| Analyse für $C_{15}H_{29}ClN_2O_2$ | | | | |
|---|---|---|---|---|
| Berechnet: | C 59,10 % | H 9,59 % | N 9,19 % | Cl 11,63 % |
| Gefunden: | C 59,05 % | H 9,69% | N 9,13 % | Cl 11,52 % |

b) N-[2-(2,2,6,6-Tetramethyl-piperidin-4-yloxy)-acetyl]-N-(2,2,6,6-tetramethyl-piperidin-4-yl)-3-methoxypropylamin
Dieses Produdukt erhält man in Analogie zu dem unter Beispiel 15 b beschriebenen Verfahren, durch Umsetzung von 30,44 g (0,1 Mol) N-(2-Chloracetyl)-N-(2,2,6,6-tetramethyl-piperidin-4yl)-3-methoxypropylamin mit 19,83 g (0,11 Mol) des Na-Alkoholats von 2,2,6,6-Tetramethyl-piperidin-4-ol in 85 ml Xylol.

Das reine Produkt erhält man durch Umkristallisieren aus n-Hexan.
Smp. 55-56 ° C

| Analyse für $C_{24}H_{47}N_3O_3$ | | | |
|---|---|---|---|
| Berechnet: | C 67,72 % | H 11,13 % | N 9,87 % |
| Gefunden: | C 67,91 % | H 11,23 % | N 9,84 % |

Beispiel 20: N-[2-(2,2,6,6-Tetramethyl-piperidin-4-yloxy)-acetyl]-N-(2,2,6,6-Tetramethyl-piperidin-4-yl)-n-octylamin

Eine Lösung von 24,9 g (0,22 Mol) Chloracetylchlorid in 30 ml Xylol wird langsam unter Rühren einer auf -10° C gekühlten von 53,6 g (0,2 Mol) N-(2,2,6,6-Tetramethyl-piperidin-4-yl)-n-octylamin in 160 ml Xylol zugetropft, wobei die Temperatur -5° C nicht übersteigen darf. Das Reaktionsgemisch wird 10 Stunden weitergerührt und die Temperatur auf Zimmertemperatur steigen gelassen. Danach wird ei wieder auf 0° C abgekühlt und eine Lösung von 8,8 g (0,22 Mol) NaOH in 50 ml Wasser innerhalb einer Stunde zugetropft. Man rührt eine Stunde bei Zimmertemperatur, man trennt die wässrige Phase ab und die organische Phase wird durch azeotrope Distillation getrocknet und eingeengt bis eine wasserfreie Lösung vorliegt. Gemäss gaschromatographischer Analyse ist das erhaltene N-(2-Chloracetyl)-N-(2,2,6,6-Tetramethyl-piperidin-4-yl)-n-octylamin zu 96,6 % rein und kann direkt für die drauffolgende Umsetzung eingesetzt werden.

Die erhaltene Lösung wird auf 100 ml eingeengt und auf 0-10° C abgekühlt. Darin wird eine zuvor durch Umsetzung von 4,6 g (0,2 Mol) Natrium mit 31,4 g (0,2 Mol) 2,2,6,6-Tetramethylpiperidin-4-ol in 150 ml wasserfreiem Xylol erhaltene Lösung des Na-Alkoholats von 2,2,6,6-Tetramethyl-piperidin-4-ol langsam, bei ständigem Rühren zugetropft. Die Temperatur wird dabei zwischen 0 und 10° C gehalten. Nach Beendigung der Zugabe rührt man 2 Stunden bei Zimmertemperatur und weitere 8 Stunden zwischen 60 und 70° C. Danach wird das Reaktionsgemisch abgekühlt und mit Wasser gewaschen, getrocknet und die flüchtigen Teile abgedampft. Der ölige Rückstand wird destilliert.
Sdp. 185° C/0,65 mbar.

| Analyse für $C_{28}H_{55}N_3O_2$ | | | |
|---|---|---|---|
| Berechnet: | C 72,21 % | H 11,90 % | N 9,02 % |
| Gefunden: | C 72,11 % | H 11,98 % | N 8,98 % |

Beispiel 21: Je 2 g der in Tabelle 2 angeführten Verbindungen und 1 g Pentaerythrit-tetrakis-3-(3,5-di-t-butyl-4-hydroxyphenyl)-propionat (Antioxydans) werden mit 1000 g Polypropylen mit einem Schmelzindex von 2,4 (®Propathen HF 18, Produkt der Imperial Chemical Industries) und 1 g Calciumstearat innig vermischt.

Das erhaltene Gemisch wird dann bei einer Temperatur von 180 bis 220° C extrudiert und zu Granulat verarbeitet, aus dem man Bänder einer Dicke von 50 µm und einer Breite von 2,5 mm herstellt. Die Arbeitsbedingungen sind:

| | |
|---|---|
| Extrudertemperatur | : 220 bis 240° C |
| Kopftemperatur | : 240° C |
| Streckverhältnis | : 1:6 |

Die erhaltenen Bänder werden auf weisser Pappe angeordnet in eines Weather-Ometer 65 WR(ASTM G 27-70) bei einer Temperatur der schwarzen Tafel vond 63° C ausgesetzt. Von Zeit zu Zeit werden Proben entnommen, an denen man die verbleibende Zugfestigkeit mittels eines Tensometers mit konstanter Geschwindigkeit misst; danach bestimmt man die zum Halbieren der ursprünglichen Zugfestigkeit erforderliche Aussetzungszeit ($T_{50}$).

Die Resultate sind in Tabelle 2 wiedergegeben.

Tabelle 2

| Stabilisator | $T_{50}$ (Stunden) |
|---|---|
| keiner | 230 |
| Verbindung von Beispiel 1 | 2600 |
| Verbindung von Beispiel 2 | 2430 |
| Verbindung von Beispiel 3 | 2850 |
| Verbindung von Beispiel 4 | 2900 |
| Verbindung von Beispiel 5 | 1640 |
| Verbindung von Beispiel 6 | 2520 |
| Verbindung von Beispiel 7 | 1760 |
| Verbindung von Beispiel 8 | 1950 |
| Verbindung von Beispiel 9 | 2070 |
| Verbindung von Beispiel 10 | 2600 |
| Verbindung von Beispiel 12 | 1720 |
| Verbindung von Beispiel 13 | 2040 |
| Verbindung von Beispiel 15 | 2880 |
| Verbindung von Beispiel 16 | 2170 |
| Verbindung von Beispiel 17 | 2540 |
| Verbindung von Beispiel 18 | 2300 |
| Verbindung von Beispiel 20 | 1860 |

Beispiel 22: Je 1,0 g der in Tabelle 3 aufgeführten Stabilisatoren, 1 g Pentaerythrit-tetrakis-3-(3,5-di-t-butyl-4-hydroxyphenyl)-proprionat, 1 g Calciumstearat, 1 g einer Pigmentpräparation (®Filofin Blue 600 der CIBA-GEIGY AG) werden in 1000 g Polypropylen mit einem Schmelzindex von 3,0 (®Propathen HF 18, der Firma Imperial Chemical Industries) innig vermischt. Das erhaltene Gemisch wird dann bei einer Temperatur zwischen 200 und 220 °C extrahiert und zu Granulat zerschnitten, aus dem man durch Formpressen bei 250 °C Platten von 2 mm Dicke erhält. Die Platten werden in einem Weather-Ometer 65 WR (ASTM G 27-70) mit einer Temperatur der schwarzen Tafel von 63 °C ausgesetzt, bis zum eben wahrnehmbaren Beschlagens der Oberfläche (chalking). Die dazu erforderliche Zeit (in Stunden) ist in Tabelle 3 als Mass für die Wirksamkeit als Stabilisator angegeben.

Tabelle 3

| Stabilisator | Zeit bis zum Beschlagen (in Stunden) |
|---|---|
| keiner | 500 |
| Verbindung von Beispiel 2 | 2200 |
| Verbindung von Beispiel 3 | 2480 |
| Verbindung von Beispiel 4 | 2660 |
| Verbindung von Beispiel 5 | 2480 |
| Verbindung von Beispiel 6 | 2310 |
| Verbindung von Beispiel 12 | 3230 |
| Verbindung von Beispiel 15 | 3650 |
| Verbindung von Beispiel 16 | 3770 |
| Verbindung von Beispiel 17 | 3300 |
| Verbindung von Beispiel 18 | 3770 |
| Verbindung von Beispiel 20 | 3230 |

**Patentansprüche**

**1.** Verbindungen der Formel I,

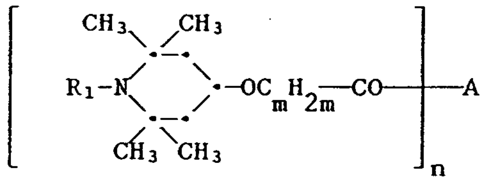

(I)

worin $R_1$ Wasserstoff, 0 , Cyanomethyl, $C_1$-$C_{12}$-Alkyl, $C_3$-$C_{12}$-Alkenyl oder Alkynyl, $C_7$-$C_{12}$-Aralkyl, Methylbenzyl, t-Butylbenzyl, Hydroxybenzyl oder $C_1$-$C_{12}$-Acyl ist, m eine ganze Zahl von 1 bis 12 und n eine ganze Zahl von 1 bis 3 bedeuten und A, wenn n = 1, eine Gruppe-N($R_2$)($R_3$) bedeutet, worin $R_2$ und $R_3$ unabhängig voneinander Wasserstoff, $C_1$-$C_{18}$-Alkyl, 2-Methoxyethyl, 2-Ethoxyethyl, 3-Methoxypropyl, 3-Ethoxypropyl, 3-Butoxypropyl, 3-Octyloxypropyl, 3-Dodecyloxypropyl, 3-Dimethylaminopropyl, 3-Diethylaminopropyl, 4-Diethylaminobutyl, $C_3$-$C_{18}$-Alkenyl, $C_5$-$C_{18}$-Cycloalkyl, Methylcyclohexyl, Trimethylcyclohexyl, $C_6$-$C_{18}$-Aryl, Methylphenyl, Dimethylphenyl, Tri-methylphenyl, t-Butylphenyl, Methoxyphenyl, Ethoxyphenyl, 3,5-Di-tert-butyl-4-hydroxyphenyl, $C_7$-$C_{18}$ Aralkyl, Methylbenzyl, Hydroxybenzyl, 3,5-Di-tert-butyl-4-hydroxybenzyl oder eine Gruppe der Formel II,

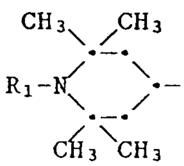

(II)

worin $R_1$ die oben angegebene Bedeutung hat, oder A Pyrrolidin-1-yl, Piperidin-1-yl, 2,2,6,6-Tetramethyl-piperidin-1-yl, Morpholin-4-yl, 4-Methyl-piperazin-1-yl, 3,3,5,5-Tetramethyl-piperazin-1-yl, Hexahydroazepin-1-yl, Azacyclotridec-1-yl oder einen Rest der Formel

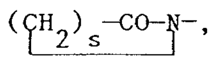

worin s eine ganze Zahl von 3 bis 11 ist, bedeutet und
A, wenn n = 2, einen Rest der Formel III, IV oder V

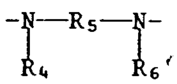 ,

(III)

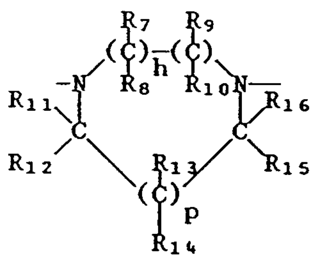

(IV)

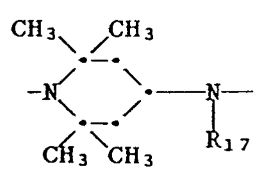

(V)

bedeutet, worin $R_4$, $R_6$ und $R_{17}$ unabhängig voneinander die bereits für $R_2$ und $R_3$ angegebene Bedeutung haben, $R_5$ $C_2$-$C_{18}$-Alkylen, 3-Oxapentan-1,5-diyl, 4-Oxaheptan-1,7-diyl, 4,9-Dioxadodecan-1,12-diyl, 4,7,10-Trioxatridecan-1,13-diyl, $C_5$-$C_{12}$-Cycloalkylen, Cyclohexylendimethylen, $C_6$-$C_{12}$-Arylen, Xylylen oder $C_7$-$C_{12}$-Aralkylen ist, die Reste $R_7$ bis $R_{16}$ unabhängig voneinander Wasserstoff oder Methyl bedeuten, $R_9$ zusammen mit $R_{10}$ und $R_{13}$ zusammen mit $R_{14}$ auch Sauerstoff bedeuten können, p und h unabhängig voneinander Null oder 1 sind, und
A, wenn n = 3, eine Gruppe der Formel VI

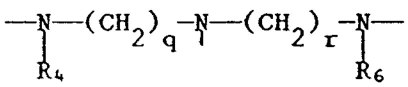

(VI)

bedeutet, worin $R_4$ und $R_6$ die oben angegebene Bedeutung haben und q und r unabhängig voneinander eine ganze Zahl von 2 bis 6 sind oder A einen Hexahydro-1,3,5-triazin-1,3,5-triyl-Rest bedeutet.

**2.** Verbindungen gemäss Anspruch 1, der Formel I, worin $R_1$ Wasserstoff, Methyl, Allyl, Benzyl oder Acetyl bedeutet, m eine Zahl von 1 bis 6 ist, $R_2$, $R_3$, $R_4$, $R_6$ und $R_{17}$ Wasserstoff, $C_1$-$C_{12}$-Alkyl, 3-($C_1$-$C_6$-Alkoxy)-propyl, $C_6$-$C_{12}$-Cycloalkyl oder eine Gruppe der Formel II worin $R_1$ die bereits in diesem Anspruch angegebene Bedeutung hat und $R_5$ $C_2$-$C_{12}$-Alkylen ist oder A als heterocyclischer Rest Piperidin-1-yl, 2,2,6,6-Tetramethyl-piperidin-1-yl, Hexahydroazepin-1-yl, Morpholin-4-yl, Piperazin-1,4-diyl oder 5,5,7-Trimethylhomopiperazin-1,4-diyl bedeutet.

**3.** Verbindungen gemäss Anspruch 1 der Formel I, worin $R_1$ Wasserstoff oder Methyl, m 1 oder 2, $R_2$, $R_3$, $R_4$, $R_6$ und $R_{17}$ $C_1$-$C_8$-Alkyl, Cyclohexyl, 2,2,6,6-Tetramethyl-piperidin-4-yl oder 1,2,2,6,6-Pentamethyl-piperidin-4-yl und $R_5$ $C_2$-$C_6$-Alkylen bedeuten oder A als heterocyclischer Rest Piperazin-1,4-diyl oder 5,5,7-Trimethylhomopiperazin-1,4-diyl ist.

**4.** Verbindungen gemäss Anspruch 1 der folgenden Formeln

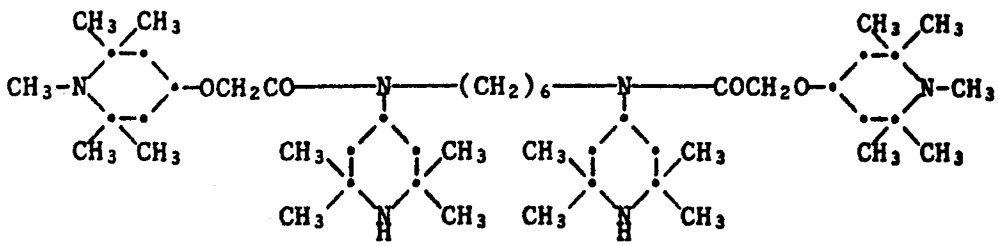

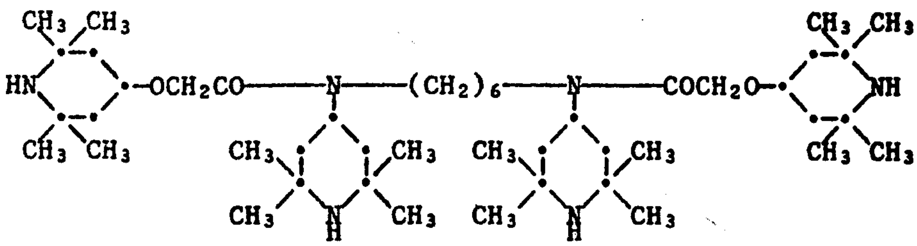

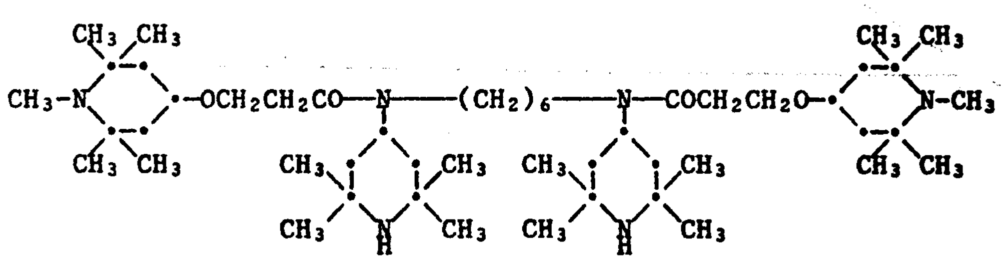

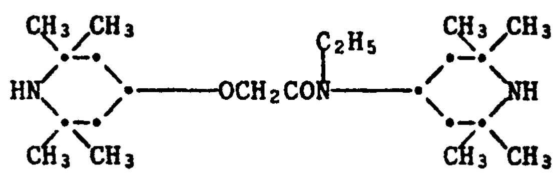

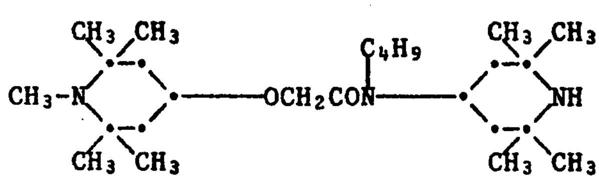

.

5. Polymerzusammensetzungen, dadurch gekennzeichnet, dass sie ein synthetisches Polymeres und eine Stabilisatorverbindung der Formel I gemäse Anspruch 1 in einer Menge zwischen 0,01 und 5 Gew.-%, bezogen auf das Gewicht des synthetischen Polymeren enthalten.

6. Zusammensetzungen nach Anspruch 5, dadurch gekennzeichnet, dass sie zusätzlich zu dem neuen Stabilisator weitere übliche Zusatzstoffe für synthetische Polymere enthalten.

7. Zusammensetzungen nach Anspruch 5, dadurch gekennzeichnet, dass als synthetische Polymeres Polyäthylen oder Polypropylen vorliegt.

8. Zusammensetzungen gemäss Anspruch 5, enthaltend ein synthetisches Polymeres und eine Stabilisatorverbindung der Formel

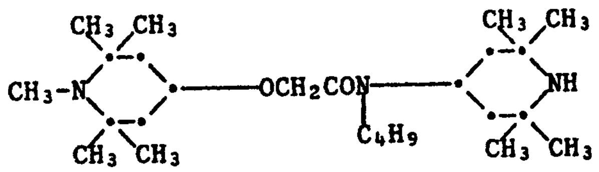 .

9. Zusammensetzungen gemäss Anspruch 5, enthaltend ein synthetisches Polymeres und eine Stabilisatorverbindung der Formel

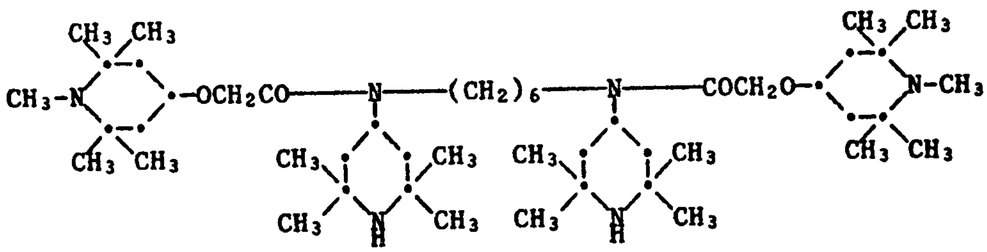 .

## Claims

1. A compound of the formula I

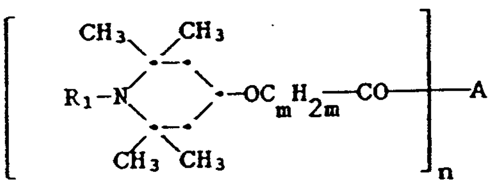

(I)

in which $R_1$ is hydrogen, O , cyanomethyl, $C_1$-$C_{12}$alkyl, $C_3$-$C_{12}$alkenyl or alkynyl, $C_7$-$C_{12}$aralkyl, methylbenzyl, t-butylbenzyl, hydroxybenzyl or $C_1$-$C_{12}$acyl, m is an integer from 1 to 12 and n is an integer from 1 to 3 and, if n = 1, A is a group -N($R_2$)($R_3$) in which $R_2$ and $R_3$ independently of one another are hydrogen, $C_1$-$C_{18}$alkyl, 2-methoxyethyl, 2-ethoxyethyl, 3-methoxypropyl, 3-ethoxypropyl, 3-butoxypropyl, 3-octyloxypropyl, 3-dodecyloxypropyl, 3-dimethylaminopropyl, 3-diethylaminopropyl, 4-diethylaminobutyl, $C_3$-$C_{18}$alkenyl, $C_5$-$C_{18}$cycloalkyl, methylcyclohexyl, trimethylcyclohexyl, $C_6$-$C_{18}$aryl, methylphenyl, dimethylphenyl, trimethylphenyl, t-butylphenyl, methoxyphenyl, ethoxyphenyl, 3,5-di-tert-butyl-4-hydroxyphenyl, $C_7$-$C_{18}$aralkyl, methylbenzyl, hydroxybenzyl, 3,5-di-tert-butyl-4-hydroxybenzyl or a group of the formula II

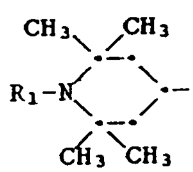

(II)

in which $R_1$ is as defined above, or A is 1-pyrrolidinyl, 1-piperidyl, 2,2,6,6-tetramethyl-1-piperidyl, 4-morpholinyl, 4-methyl-1-piperazinyl,3,3,5,5-tetramethyl-1-piperazinyl, hexahydroazepin-1-yl,

azacyclotridec-1-yl or a radical of the formula

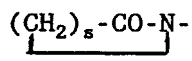

in which s is an integer from 3 to 11 and, if n = 2, A is a radical of the formula III, IV or V

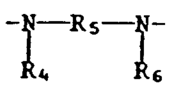

(III)

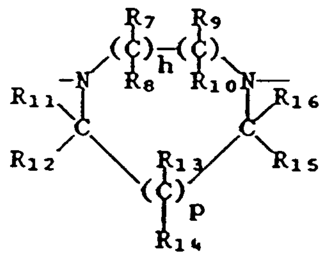

(IV)

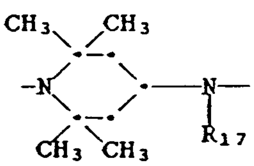

(V)

in which $R_4$, $R_6$, and $R_{17}$ independently of one another are as already defined for $R_2$ and $R_3$, $R_5$ is $C_2$-$C_{18}$alkylene, 3-oxapentane-1,5-diyl, 4-oxaheptane-1,7-diyl, 4,9-dioxadodecane-1,12-diyl, 4,7,10-trioxatridecane-1,13-diyl, $C_5$-$C_{12}$cycloalkylene, cyclohexylenedimethylene, $C_6$-$C_{12}$arylene, xylylene or $C_7$-$C_{12}$aralkylene, the radicals $R_7$ to $R_{16}$ independently of one another are hydrogen or methyl, $R_9$, together with $R_{10}$ and $R_{13}$ together with $R_{14}$ can also be oxygen, p and h independently of one another are zero or 1 and, if n = 3, A is a group of the formula VI

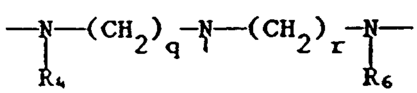

(VI)

in which $R_4$ and $R_6$ are as defined above and q and r independently of one another are integers from 2 to 6, or A is a hexahydro-1,3,5-triazine-1,3,5-triyl radical.

**2.** A compound according to claim 1, of the formula I in which $R_1$ is hydrogen, methyl, allyl, benzyl or acetyl, m is a number from 1 to 6, $R_2$, $R_3$, $R_4$, $R_6$ and $R_{17}$ are hydrogen, $C_1$-$C_{12}$alkyl, 3-($C_1$-$C_6$alkoxy)-propyl, $C_6$-$C_{12}$cycloalkyl or a group of the formula II in which $R_1$ is as already defined in this claim and $R_5$ is $C_2$-$C_{12}$alkylene or A as a heterocyclic radical is 1-piperidyl, 2,2,6,6-tetramethyl-1-piperidyl, hexahydroazepin-1-yl, 4-morpholinyl, piperazine-1,4-diyl or 5,5,7-trimethylhomopiperazine-1,4-diyl.

**3.** A compound according to claim 1, of the formula I in which $R_1$ is hydrogen or methyl, m is 1 or 2, $R_2$, $R_3$, $R_4$, $R_6$ and $R_{17}$ are $C_1$-$C_8$alkyl, cyclohexyl, 2,2,6,6-tetramethyl-4-piperidyl, or 1,2,2,6,6-pentamethyl-4-piperidyl and $R_5$ is $C_2$-$C_6$alkylene or A as a heterocyclic radical is piperazine-1,4-diyl or 5,5,7-trimethylhomopiperazine-1,4-diyl.

**4.** A compound according to claim 1, of one of the following formulae:

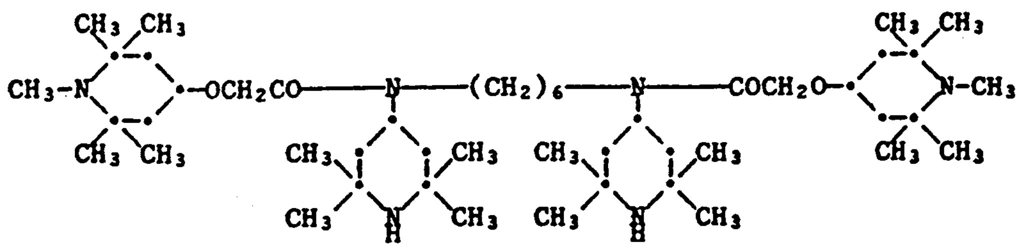

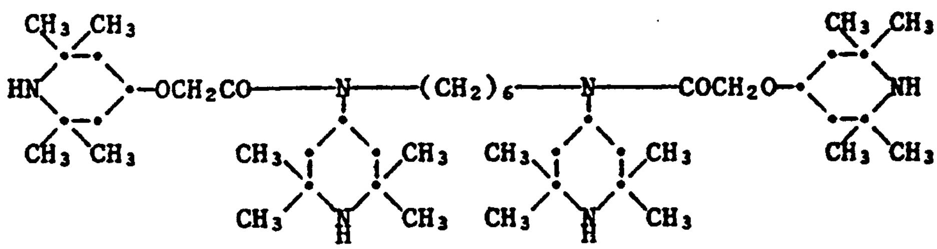

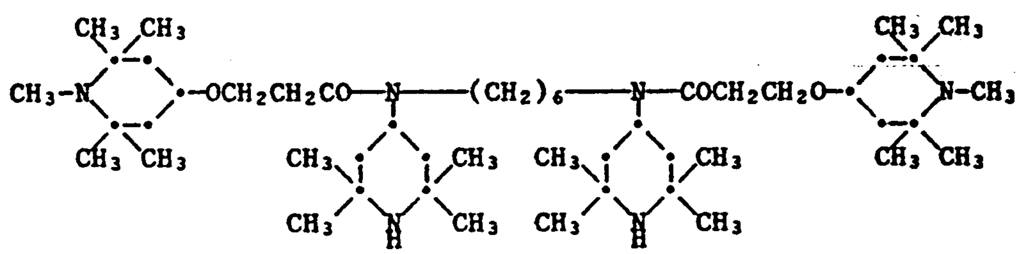

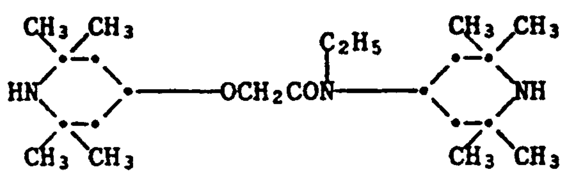

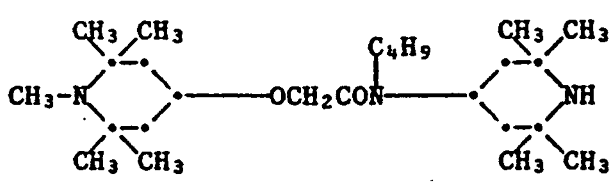

.

5. A polymer composition comprising a synthetic polymer and a stabiliser compound of the formula I according to claim 1 in a quantity of between 0.01 and 5 % by weight, relative to the weight of the synthetic polymer.

6. A composition according to claim 5, which, in addition to the novel stabiliser, comprises other conventional additives for synthetic polymers.

7. A composition according to claim 5, wherein the synthetic polymer is polyethylene or polypropylene.

8. A composition according to claim 5, comprising a synthetic polymer and a stabiliser compound of the formula

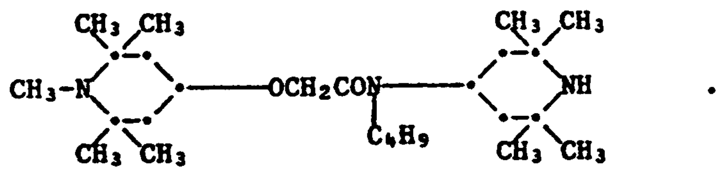

9. A composition according to claim 5, comprising a synthetic polymer and a stabiliser compound of the formula

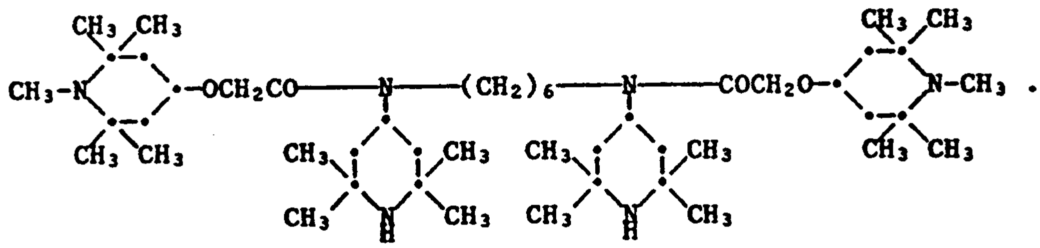

## Revendications

1. Composés répondant à la formule I :

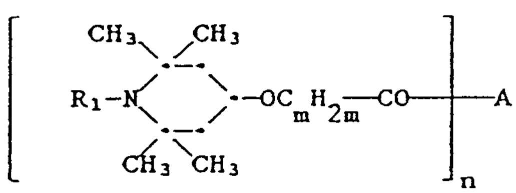

(I)

dans laquelle

$R^1$ représente l'hydrogène, $O^-$, un cyanométhyle, un alkyle en $C_1$-$C_{12}$, un alcényle en $C_3$-$C_{12}$, un alcynyle en $C_3$-$C_{12}$, un aralkyle en $C_7$-$C_{12}$, un méthylbenzyle, un tert-butyl-benzyle, un hydroxybenzyle ou un acyle en $C_1$ -$C_{12}$,

m désigne un nombre entier de 1 à 12,

n désigne un nombre entier de 1 à 3 et

A représente :

- lorsque n est égal à 1, un radical -N($R_2$) ($R_3$) dans lequel $R_2$ et $R_3$ représentent chacun, indépendamment l'un de l'autre, l'hydrogène, un alkyle en $C_1$-$C_{18}$, un 2-méthoxy-éthyle, un 2-éthoxy-éthyle, un 3-méthoxy-propyle, un 3-éthoxy-propyle, un 3-butoxy-propyle, un 3-octyloxy-propyle, un 3-dodécyloxy-propyle, un 3-diméthylamino-propyle, un 3-diéthylaminopropyle, un 4-diéthylaminobutyle, un alcényle en $C_3$-$C_{18}$, un cycloalkyle en $C_5$-$C_{18}$, un méthylcyclohexyle, un triméthylcyclohexyle, un aryle en $C_6$-$C_{18}$, un méthylphényle, un diméthylphényle, un triméthyl-phényle, un tert-butylphényle, un méthoxyphényle, un éthoxyphényle, un 3,5-di-tert-butyl-4-hydroxyphényle, un aralkyle en $C_7$-$C_{18}$, un méthylbenzyle, un hydroxybenzyle, un 3,5-di-tert-butyl-4-hydroxybenzyle ou un radical de formule II :

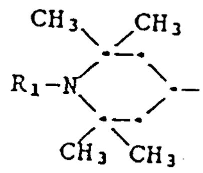

(II)

(dans lequel $R_1$ a la signification qui lui a été donnée ci-dessus), un radical pyrrolidine-1-yle, pipéridine-1-yle, 2,2,6,6-tétraméthyl-pipéridine-1-yle, morpholine-4-yle, 4-méthyl-pipérazine-1-yle, 3,3,5,5-tétraméthyl-pipérazine-1-yle, hexahydroazépine-1-yle ou aza-cyclotridéc-1-yle ou un radical

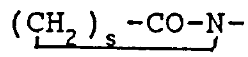

dans lequel s désigne un nombre entier de 3 à 11,

- lorque n est égal à 2, un radical répondant à l'une des formules III, IV et V

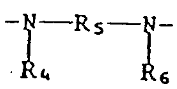

(III)

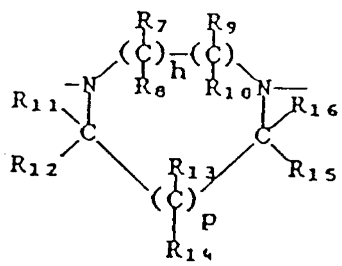

(IV)

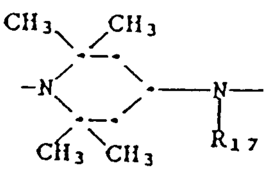

(V)

dans lesquelles $R_4$, $R_6$ et $R_{17}$ ont chacun, indépendamment l'un de l'autre, les significations qui ont été données pour $R_2$ et $R_3$, $R_5$ représente un alkylène en $C_2$-$C_{18}$, un 3-oxa-pentane-1,5- diyle, un 4-oxa-heptane-1,7-diyle, un 4,9-dioxa-dodécane-1,12-diyle, un 4,7,10-trioxa-tridécane-1,13-diyle, un cycloalkylène en $C_5$-$C_{12}$, un cyclohexylène-diméthylène, un arylène en $C_6$-$C_{12}$, un xylylène ou un aralkylène en $C_7$-$C_{12}$, $R_7$ à $R_{16}$ représentent chacun, indépendamment l'un de l'autre, l'hydrogène ou un méthyle, $R_9$ associé à $R_{10}$, et $R_{13}$ associé à $R_{14}$, pouvant également représenter l'oxygène, et p et h sont égaux chacun, indépendamment l'un de l'autre, à 0 ou à 1, et

- lorsque n est égal à 3, un radical répondant à la formule VI :

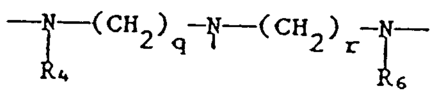

(VI)

(dans laquelle $R_4$ et $R_6$ ont les significations qui leur ont été données ci-dessus, et q et r représentent chacun, indépendamment l'un de l'autre, un nombre entier de 2 à 6) ou un radical hexahydro-1,3,5-triazine-1,3,5-triyle.

**2.** Composés de formule I selon la revendication 1 dans lesquels $R_1$ représente l'hydrogène ou un radical méthyle, allyle, benzyle ou acétyle, m représente un nombre de 1 à 6, $R_2$, $R_3$, $R_4$ ,$R_6$ et $R_{17}$ représentent chacun l'hydrogène, un alkyle en $C_1$-$C_{12}$, un 3-($C_1$-$C_6$-alcoxy)-propyle, un cycloalkyle en $C_6$-$C_{12}$ ou un radical de formule II dans lequel $R_1$ a la signification qui lui a déjà été donnée dans cette revendication, et $R_5$ représente un alkylène en $C_2$-$C_{12}$, ou A, en tant que radical hétérocyclique, représente un radical pipéridine-1-yle, 2,2,6,6-tétraméthyl-pipéridine-1-yle, hexahydroazépine-1-yle, morpholine-4-yle, pipérazine-1-4- diyle ou 5,5,7-triméthyl-homopipérazine-1,4-diyle.

**3.** Composés de formule I selon la revendication 1 dans lesquels $R_1$ représente l'hydrogène ou un méthyle, m est égal à 1 ou à 2, $R_2$, $R_3$, $R_4$, $R_6$ et $R_{17}$ représentent chacun un alkyle en $C_1$-$C_8$, un cyclohexyle, un 2,2,6,6-tétraméthyl-pipéridine-4-yle ou un 1,2,2,6,6-pentaméthyl-pipéridine-4-yle et $R_5$ représente un alkylène en $C_2$-$C_6$ ou A représente, en tant que radical hétérocyclique, un radical pipérazine-1,4-diyle ou 5,5,7-triméthyl-homopipérazine-1,4-diyle.

**4.** Composés selon la revendication 1 qui répondent aux formules suivantes :

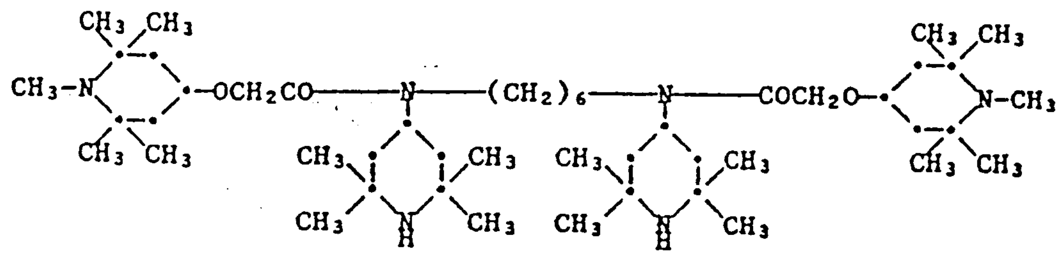

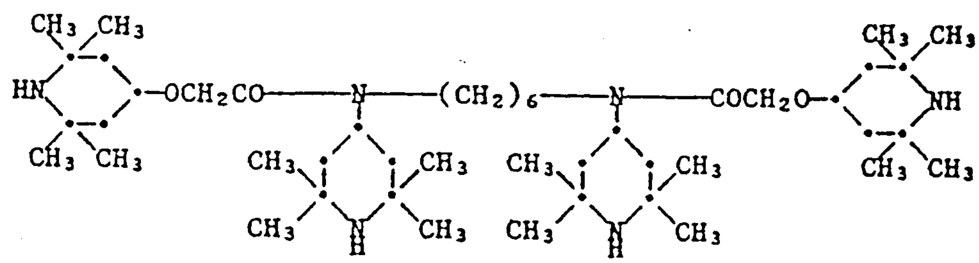

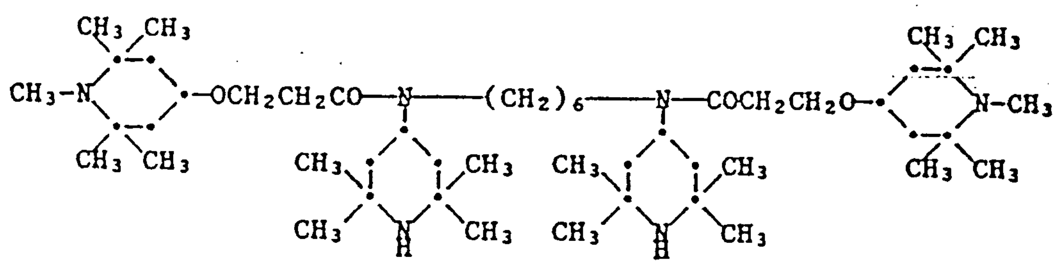

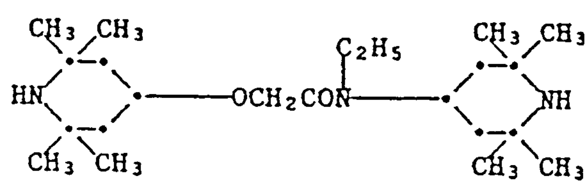

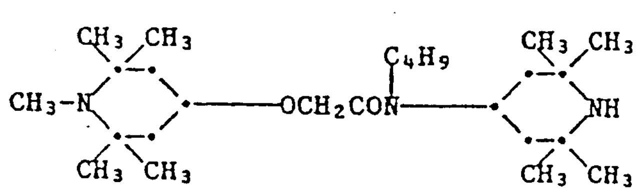

**5.** Compositions à base de polymères, compositions caractérisées en ce qu'elles contiennent un polymère synthétique et un stabilisant de formule I selon la revendication 1 en une quantité comprise entre 0,01 et 5 % en poids par rapport au poids du polymère synthétique.

**6.** Compositions selon la revendication 5 caractérisées en ce qu'elles contiennent, en plus du nouveau stabilisant, des additifs usuels pour polymères synthétiques.

7. Compositions selon la revendication 5 caractérisées en ce qu'elles contiennent, comme polymère synthétique du polyéthylène ou du polypropylène.

8. Compositions selon la revendication 5 qui contiennent un polymère synthétique et un stabilisant de formule :

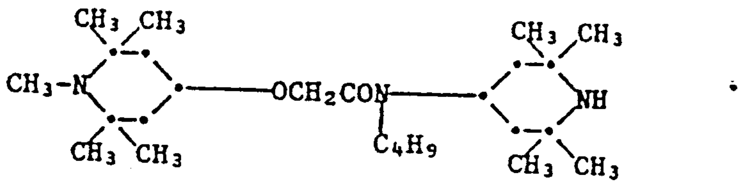

9. Compositions selon la revendication 5 qui contiennent un polymère synthétique et un composé stabilisant de formule :